# EUROPEAN PATENT SPECIFICATION

(11) **EP 0 521 578 B1**
(45) Date of publication and mention of the grant of the patent: **16.04.1997**
(21) Application number: 92202003.7
(22) Date of filing: 01.07.1992
(51) Int. Cl.: C07C 67/38, C07C 69/54

(54) **Continuous process for the carbonylation of acetylenes**
Kontinuierverfahren zur Carbonylierung von Acetylenen
Procédé continu de carbonylation d'acétylènes

(30) Priority: 03.07.1991 GB 9114327
(43) Date of publication of application: 07.01.1993
(73) Proprietor: SHELL INTERNATIONALE RESEARCH MAATSCHAPPIJ B.V., 2596 HR Den Haag (NL)
(72) Inventor: Scheffer, Hendrik Jan, NL-1031 CM Amsterdam (NL); Van der Beek, Douwe Christiaan, NL-2596 HR The Hague (NL)

(56) References cited:
- EP-A- 0 392 601

## Description

This invention relates to a continuous process for the carbonylation of an acetylenically unsaturated compound by reaction with carbon monoxide and a nucleophilic compound having a mobile hydrogen atom in the presence of a carbonylation catalyst.

Methyl methacrylate is prepared in industry mainly by the so-called acetone cyanohydrin process. This process presents disadvantages in that large quantities of waste sulphuric acid and ammonium bisulphate are produced, which have to be discharged or worked up for reuse, and in that another waste material, HCN, is highly toxic and pressures against its storage and transportation are increasing (cf. T. Haeberle and G. Emig in Chem. Eng. Technol. 11, 6 (1988) 392-402). As concerns about the environment have increased, considerable research has been devoted to finding alternative processes which do not present these disadvantages.

One possible alternative process described in 1964 by Y. Sakakibira in Bull. Chem. Soc. Japan 37, 11 (1964) 1601-1609, comprises the reaction of propyne with carbon monoxide and an alkanol in the presence of a carbonylation catalyst. Although this process has now been known for a long time, and has attracted a considerable amount of interest, it has never been commercialised.

A factor inhibiting the commercial exploitation of the carbonylation process has been the unavailability of large quantities of a suitable low-priced propyne feed, and only recently a process for overcoming this drawback was proposed in EP-A-392601, according to which a propyne feed is used which has been obtained by selectively removing propadiene from a C₃-mixture comprising a mixture of propyne and propadiene that has been obtained from an ethene cracker, a catalytic cracker or an LPG-dehydrogenation process.

EP-A-392,601 further describes a process for the carbonylation of propyne in which a carbonylation catalyst is charged to a reactor, and propyne, methanol and carbon monoxide are also supplied to the reactor. The reaction mixture is allowed to react and after the reaction, the reactor effluent is flashed and stripped of unreacted gases. Unreacted propyne can be scrubbed out with methanol and recycled to the reactor. This process is illustrated by some batch-wise examples.

For economic and environmental reasons it remains desirable to further maximise the consumption of acetylenically unsaturated compound on the basis of the carbonylation product produced. In their efforts to design an economically viable process, the present inventors encountered a problem specific to acetylenically unsaturated compounds relating to their thermally instable and therefore hazardous nature. It was initially contemplated having the reaction to proceed in a reactor to about 70% conversion per pass of the acetylenically unsaturated compound in order to maintain an acceptably high space velocity. It appeared, however, that the intended recycle of unreacted acetylenically unsaturated compound required a too high investment in terms of equipment for the lay-out to be economically viable, since a straightforward compression of the effluent gases appeared to be precluded by heat evolution and according safety restrictions.

Upon extensive further study and thought the inventors conceived and effected an unexpected solution to the problem indicated.

Accordingly the present invention provides a process according to the preamble of this specification, which process comprises the steps of:
a) charging said carbonylation catalyst to a reaction zone;
b) supplying a fresh feed containing said acetylenically unsaturated compound, said nucleophilic compound as well as carbon monoxide to said reaction zone;
c) passing the feed through the reaction zone under carbonylating conditions of pressure and temperature such that a degree of conversion, based on the acetylenically unsaturated compound, of at least 80% is effected;
d) separating a stream containing unreacted acetylenically unsaturated compound from the effluent of the reaction zone; and
e) recycling the unreacted acetylenically unsaturated compound content of said separated stream to the reaction zone.

Surprisingly, it was found that in the process according to the invention improved overall conversions of the acetylenically unsaturated compound into the desired carbonylation product above 90%, even above 95% can be obtained with lower net costs of equipment and/or utilities than when conducting the process either at moderate conversion per pass and large recycle, or at high conversion without recycle.

The acetylenically unsaturated compounds to be carbonylated in the present process include alkynes, in particular 1-alkynes, which may be substituted or unsubstituted and may comprise up to 6 carbon atoms, in particular alkynes which are separated from the reactor effluent in gaseous form, for example ethyne, chloroethyne, propyne, 3,3,3-trifluoropropyne, and 1-butyne. Propyne is preferred.

Suitable nucleophilic compounds having a mobile hydrogen atom include hydroxy compounds, such as water, alcohols and carboxylic acids, amines and thiols, which may be aliphatic, cycloaliphatic or aromatic, preferably contain not more than 20 carbon atoms, and may have more than one mobile hydrogen atom. Examples of suitable alcohols include methanol, ethanol, propanol, isopropanol, isobutanol, n-butanol, t-butanol, stearyl alcohol, phenol, ethylene glycol and glycerol. Examples of suitable carboxylic acids include acetic acid and propionic acid. Examples of suitable amines include aniline and n-butylamine. Examples of suitable thiols include ethanethiol and 1-propanethiol.

Preferably, the present process is used for preparing an alkyl methacrylate, such as methyl methacrylate, by reaction of propyne, an alkanol and carbon monoxide.

The carbonylation catalyst used in the process according to the invention may be any catalyst having activity for the carbonylation of acetylenically unsaturated compounds, and may be heterogeneous or homogeneous.

Preferably, the carbonylation catalyst is based on a composition of a Group VIII (e.g. palladium) compound, a ligand (e.g. a phosphine), and an anion of a Broensted acid (from a salt, ester anhydride or acid, and preferably weakly or non-coordinating). A particularly preferred example of such a catalyst is based on a composition of a palladium (II) compound, an organic phosphine of formula PR₃ in which each R independently stands for an optionally substituted hydrocarbyl or heterocyclic group, and a non-hydrohalogenic Broensted acid having a pKa < 2.

A hydrocarbyl group in an optionally substituted hydrocarbyl group is preferably an alkyl group, for example a C₁₋₆ alkyl group, such as methyl, ethyl, propyl, isopropyl, n-butyl, isobutyl or t-butyl, a cycloalkyl group, e.g. cyclohexyl, or an aryl group such as phenyl or naphthyl. Two R-groups may alternatively represent an optionally substituted alkylene chain.

A heterocyclic group in an optionally substituted heterocyclic group is preferably an aromatic group having an imino nitrogen, for example a pyridyl, pyrazinyl, quinolyl, isoquinolyl, pyrimidinyl, pyridazinyl, cinnolinyl, triazinyl, quinoxalinyl or quinazolinyl group. An imino nitrogen atom in an aromatic group having an imino nitrogen is preferably connected to phosphorus through a single bridging carbon atom, as for example in 2-pyridyl.

A non-hydrohalogenic Broensted acid may be, for example, sulphuric acid, a sulphonic acid such as p-toluenesulphonic acid, naphthalenesulphonic acid, trifluoromethanesulphonic acid, methanesulphonic acid or a sulphonated ion exchange resin; a phosphonic acid such as benzenephosphonic acid; a carboxylic acid such as trifluoroacetic acid, a perhalic acid such as perchloric acid, fluorosilicic acid, HBF₄, HPF₆ or HSbF₆.

Examples of such catalysts are mentioned in EP-A-186228 and EP-A-271144, e.g. combinations of (a) palladium acetate, (b) triphenylphosphine or diphenyl-2-pyridylphosphine, and (c) p-toluenesulphonic or trifluoroacetic acid. When the catalyst is a Group VIII metal catalyst, it is preferred that the catalyst has a conversion activity of at least 1000, more preferably at least 10,000 mol of acetylenically unsaturated compound/gram atom of catalytic metal/hour.

The carbonylation step of the present process is effected in a reaction zone in which carbonylating conditions of temperature and pressure prevail. Preferred temperatures are in the range of from 20 to 200 °C, more preferably 20 to 80 °C. Preferred pressures are in the range of from 5 to 70 bar.

The technological features of the present process will be further described with reference to propyne as the preferred acetylenically unsaturated compound feed and methanol as the preferred mobile hydrogen-comprising nucleophilic compound feed.

The reaction zone may be constituted by a single pressure container, which is operated at a LHSV (Liquid Hour Space Velocity) appropriate for effecting a degree of conversion of propyne of 80% or higher. However, this set-up may not be preferred, since at economic catalyst concentrations and moderate temperature and pressure conditions relatively low LHSV's are required for the higher order carbonylation reaction to proceed to conversions of at least 80%. Alternatively, the reaction zone may be constituted by a plug flow reactor, which allows for high conversion at higher LHSV's, but provides limited facilities for handling the gas/liquid reaction mixture and controlling the generation of heat during the reaction. Preferably, the reaction zone is constituted by a plurality, say three, of consecutive pressure containers. By passing the reaction mixture through the consecutive containers with optional additional supply of carbon monoxide and/or catalyst composition, high reaction rates in at least the first and second container can be maintained, whereas the effluent of the last container will comprise low proportions of the precursors. Thus, degrees of conversion of at least 80%, more preferably of at least 90%, can be readily achieved. The individual pressure containers can be of any known, optionally modified, design, for example provided with stirring means or with a packing, depending on the use of homogeneous or heterogeneous catalyst, and be equipped with any required inlet or outlet and control means, within the skills of the relevant technologist.

The carbonylation catalyst is charged to the reaction zone either continuously together with the feed, and accordingly continuously withdrawn, for example, when being a homogeneous catalyst, or in advance for fixed residence in the reaction zone, for example, when being a heterogeneous catalyst. For regeneration or replacement purposes, the heterogeneous catalyst packing may be removed entirely at intervals or continuously in part. When the catalyst is a homogeneous catalyst, it is conveniently dissolved in one of the precursors, for example methanol, and brought together with the liquid reactants into a mixing device, and then fed into the reaction zone. The catalyst may be fed separately, or in portions, for example to the several pressure containers constituting a reaction zone. Some of the catalyst components, such as the ligand and/or the Broensted acid anion may be fed to various locations in the reaction zone, while the Group VIII metal compound is added together with the fresh feed of the liquid reactants.

The propyne and methanol contained in the fresh feed to the reaction zone, irrespective of any recycle streams, will approximately be consumed according to the reaction stoichiometry, which is equimolar for the present carbonylation reaction. It is therefore preferred that the fresh feed comprises propyne and methanol in substantial equimolar ratio, thus avoiding the discharge of any substantial waste stream of excess propyne or methanol from the process. Preferably, the molar ratio of the acetylenically unsaturated compound, such as propyne, to the nucleophilic compound having a mobile hydrogen atom, such as methanol, is between 0.8 : 1 and 1 : 0.8, more preferably between 0.9 : 1 and 1 : 0.9.

The carbon monoxide may be fed to the reaction zone separately or together with the liquid feed. Being gaseous, it should be supplied under a pressure at least equal to the pressure prevailing in the reaction zone.

The effluent of the reaction zone will comprise methyl methacrylate product, unreacted propyne, methanol and carbon monoxide, the catalyst residue and minor amounts of side product such as dimethylketone and methyl crotonate. It is a feature of the invention that at least part of the residual propyne is separated from the effluent and recycled to the reaction zone, despite the fact that the carbonylation reaction is driven to near completion. Any method suitable for separating the propyne from the reaction effluent, such as selective extraction, flashing or distilling, can be practised within the scope of the invention. It should be borne in mind, however, that the reaction zone is operated at elevated pressure. Therefore, for recycling the propyne contained in the recycle stream, should be either compressed as a gas or be condensed to the liquid state, and pressurised to the same or higher pressure in order to enable resupply to the reaction zone.

According to a first useful embodiment of the invention, the reaction effluent is flashed and stripped of unreacted gases to provide a gas stream and a liquid stream. The gas stream is chilled (to about - 20 °C) to condense part of the propyne for return to the liquid feed.

According to a second useful embodiment of the invention, the liquid stream obtained after flashing is fed to a distillation column as a first step of the purification procedure for the methyl methacrylate product, and the light ends of this distillation column are compressed and combined with the gas stream of the reactor flash for further condensation and recycle to the liquid feed.

According to a third useful embodiment of the invention, the gaseous remainder after separation of the condensable components for recycle to the liquid feed, is compressed from the reactor flash pressure to the reaction pressure for recycle of additional propyne values in gaseous form to the reaction zone. The compression is effected stepwise with intermittent cooling for discharging the evolving heat in view of the thermal instability of the propyne.

Each of the above embodiments surprisingly appeared to be advantageous in that the expenditure of the recycle of small propyne waste streams is outweighed by an increase of methyl methacrylate product yield.

According to a fourth embodiment of the invention, which is preferred, at least part of the propyne is separated for recycling by stripping the effluent of the reaction zone with at least part of the fresh carbon monoxide feed to the process. This embodiment is particularly advantageous in that a pressure drop as occurring when flashing, is avoided, so that the propyne recovered can directly be recycled to the reaction zone in gaseous form. Moreover, dilution with the carbon monoxide feed renders the propyne partial pressure attractively low for safety considerations.

In this preferred embodiment, the reactor effluent can be fed to a stripping zone, for example constituted by a stripping column provided with a packing, trays or the like, for contacting with the fresh carbon monoxide feed. Preferably, the reactor effluent is contacted countercurrently with the carbon monoxide stream. The pressure and temperature for operation of the stripping zone are not critical. It is preferred, that the stripping zone is operated at the pressure of the carbon monoxide feed, more preferably at a pressure in the range of from 5 to 20 bar. Thus, optimum benefits of the invention are achieved. The temperature of the stripping zone is conveniently controlled by the temperatures of the reactor effluent and the carbon monoxide feed. A carbon monoxide feed of ambient temperature may thus be preheated. However, external temperature control by heating or cooling means is not excluded. Preferred temperatures for operation of the stripping zone are in the range of from 20 to 100 °C. By stripping with the carbon monoxide feed about 40 to 70% of the unreacted propyne contained in the reactor effluent may be separated for recycling. As a rule, lower pressures and higher temperatures in the stripping zone will tend to increase the rate of separation and recycle of the unreacted propyne.

Within the context of the present invention, any of the features of the above embodiments may be applied in combination for further increase of the rate of recycle of unreacted propyne.

The liquid fraction of the reactor effluent, which contains light ends, product, catalyst residues, and heavy ends is typically sent to a distillation section for rectification. The distillation section may comprise a plurality of distillation units and any conducts, compressors and/or condensers required for handling the various streams generated. Further constructional details, such as valves, metering devices, temperature controls, and the like may be present as will be appreciated by the skilled man.

In the distillation section, various streams are generated which include a purified product stream, and light and heavy ends bleed streams containing the low boiling and high boiling impurities removed. The heavy ends bleed stream will usually comprise the catalyst residue, and may be disposed of in a responsible way, optionally after additional methyl methacrylate recovery using a heavy ends stripper. The light ends bleed stream, being depleted of propyne in accordance with the invention to a desired extent, may be flared or disposed of in any other acceptable way. Any propyne-containing streams generated in the distillation section and intended for recycle to the reaction zone, may be gaseous or liquid. Liquid recycle streams can readily be brought to a higher pressure for combination with the reaction liquid or any of the liquid feed streams. Gaseous recycle streams will have to be compressed to at least the reaction pressure, using a plurality of compression cycles, if necessary, for restricting the compression ratio per cycle in view of safety considerations. Condensers may be used for liquifying propyne values from gaseous streams, if desired.

A further stream may be generated, which comprises an azeotrope, for example of methyl methacrylate and methanol, and is preferably recycled to the reaction zone. This will, of course, improve the overall yield of methyl methacrylate product. Moreover, under steady state operating conditions of the present continuous process, this will increase the molar ratio between methanol and propyne present in the reaction zone to above the approximately equimolar ratio in the fresh feed. As a result, depletion of the methanol reaction component in the higher order carbonylation reaction is attenuated, so that the required degree of conversion of 80% can more readily be achieved.

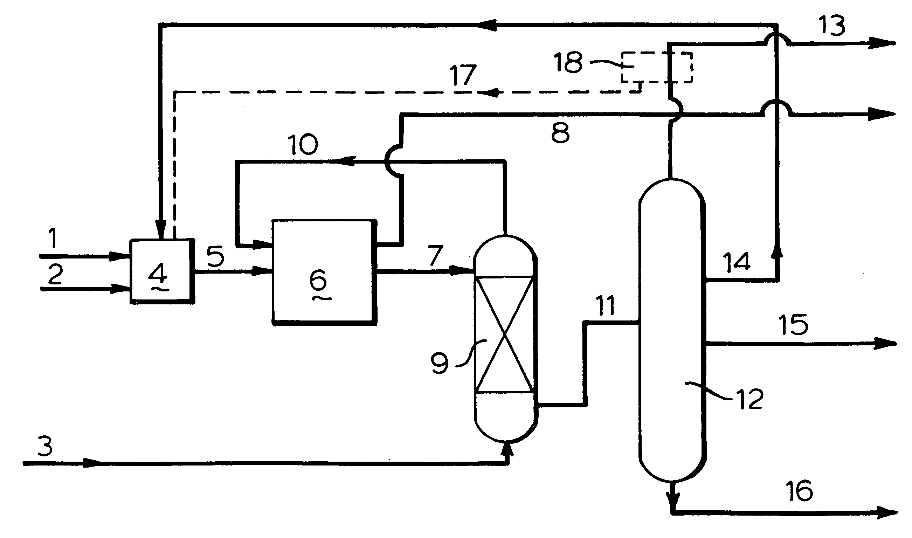

The invention will now be illustrated in more detail by the following Examples. Example 1 illustrates a process in which the effluent of the reaction zone is stripped with the fresh carbon monoxide feed according to the schematic flow scheme of the attached Figure. In this Figure, line 1 supplies propyne and methanol, line 2 catalyst composition, and line 3 carbon monoxide to the process. The liquid streams through the lines 1 and 2 are mixed in a mixing device 4, and forwarded through line 5 to a reaction zone 6, which may be constituted by a plurality of individual reactors. The effluent of the reaction zone 6 is forwarded through line 7 to a stripping zone 9. A line 8 provides for a bleed of the gaseous content of the reaction zone for removing gaseous inerts from the process. In the stripping zone 9, the reaction effluent is countercurrently contacted with the fresh carbon monoxide feed supplied through line 3. The carbon monoxide feed-containing propyne stripped from the reaction effluent, leaves the stripping zone 9 through line 10, and is supplied to the reaction zone 6. The stripped effluent is forwarded through line 11 to a distillation section 12, which may be constituted by a plurality of distillation units. In the distillation section 12, the stripped reaction effluent is fractionated into light ends leaving through line 13, an azeotrope of methyl methacrylate and methanol leaving through line 14, methyl methacrylate product leaving through line 15, and heavy ends leaving through line 16. The methyl methacrylate/methanol azeotrope is recycled through line 14 to the mixing device 4.

Example 2 illustrates a process which is similar to the process of the Figure, but additionally comprises the feature of propyne recovery from the light ends leaving through line 13 by including a condensation zone 18 (represented by dashes), and recycling of condensed propyne through (dashed) line 17 to the mixing device 4. Example 3 illustrates a process in which the carbonylation reaction is conducted to a degree of propyne conversion per pass of about 70 % and large streams of unreacted precursors have to be recycled for achieving an acceptable rate of consumption of the precursors. Example 3 is outside the scope of the invention.

### Example 1

A fresh feed consisting of 2003 kg/hr propyne, 1755 kg/hr methanol and a catalyst composition comprising palladium acetate, diphenyl(6-methyl-2-pyridyl)phosphine and p-toluenesulphonic acid in a concentration of 0.01 gram atom Pd/mol propyne is continuously introduced into the mixing device of a process illustrated in the Figure. The reaction zone comprises three consecutive pressure containers and is operated at a temperature of 45 °C and a pressure of 11 barabs. The liquid reaction phase passes the pressure containers with a LHSV of about 0.27 l/l/hr. From the gas cap of the last pressure container a reactor gas bleed stream comprising 2.3 kg/hr propyne, 58.1 kg/hr carbon monoxide, 2.1 kg/hr methanol and 2.6 kg/hr methyl methacrylate besides inerts such as nitrogen and hydrogen, is bled from the process.

The liquid reaction product comprising 4.9% of the propyne unconverted, is forwarded to a stripping column and countercurrently stripped therein with a carbon monoxide feed of 1451 kg/hr at a temperature of 55 °C and a pressure of 12 barabs. The carbon monoxide stream containing stripped-off propyne is introduced into the liquid phase of the reaction. The liquid effluent of the stripping column is flashed and fed to a first distillation column to separate a gaseous light ends bleed stream over the top of the column comprising 46.0 kg/hr propyne, 19.1 kg/hr carbon monoxide, 188.5 kg/hr methanol and 69.0 kg/hr methyl methacrylate, besides side products such as dimethylketone, which are bled from the process. A methyl methacrylate/methanol azeotrope, comprising impurities as water, ethanol and dimethoxypropane is separated as a liquid stream also over the top of this column and fed to a second distillation column to remove the impurities over the bottom, also comprising 49.8 kg/hr methyl methacrylate and 0.3 kg/hr methanol. The liquid methyl methacrylate/methanol azeotrope is recycled to the mixing device.

The liquid bottom stream of the first distillation column is fed to a third distillation column. Over the top of the third distillation column a product stream of 4707 kg/hr pure methyl methacrylate is obtained. A heavy ends bottom stream comprising 19.0 kg/hr methyl methacrylate, 38.5 kg/hr methyl crotonate besides catalyst residues and remaining heavy ends, is bled from the process.

The overall conversion of propyne in the process is 97.6%, and 94.0% of the starting propyne is converted into methyl methacrylate product. Per 1000 kg of methyl methacrylate product, 10.3 kg of propyne, 16.4 kg of carbon monoxide and 40.6 kg of methanol are lost, primarily because of the bleed streams required for withdrawal of inerts, side products and catalysts residues.

### Example 2

In this Example, a feed of 2003 kg/hr propyne, 1575 kg/hr methanol and 1451 kg/hr carbon monoxide is processed in essentially the same way as described in Example 1 with the difference that the light ends bleed stream of 330.3 kg/hr at a pressure of 0.4 barabs is partially condensed at 0 °C in a first condenser. The obtained condensate, comprising 13% of the propyne and 96.5% of the methanol contained in the light ends bleed stream is recycled to the mixing device, whereas the gaseous remainder is bled as a depleted light ends stream, comprising 42.9 kg/hr propyne, 18.8 kg/hr carbon monoxide, 6.5 kg/hr methanol and 2.2 kg/hr methyl methacrylate.

In this Example, the reactor gas bleed stream comprises 2.4 kg/hr propyne, 56.2 kg/hr carbon monoxide, 2.1 kg/hr methanol and 2.5 kg/hr methyl methacrylate. The bottom stream of the second distillation column comprises 0.3 kg/hr methanol, 50.6 kg/hr methyl methacrylate besides impurities as water, ethanol and dimethoxypropane.

A product stream of 4779 kg/hr pure methyl methacrylate is obtained, while the heavy ends bottom stream comprises 19.0 kg/hr of methyl methacrylate, 38.6 kg/hr of methyl crotonate, besides catalyst residues and remaining heavy ends.

The overall conversion of propyne in the process is 97.7%, and 95.5% of the starting propyne is converted into methyl methacrylate product. Per 1000 kg of methyl methacrylate product, 9.5 kg of propyne, 15.7 kg of carbon monoxide and 1.9 kg of methanol are lost.

### Comparative example

A fresh feed of 2003 kg/hr propyne, 1402 kg/hr carbon monoxide and 1663 kg/hr methanol, together with the same catalyst activity as in Example 1 is introduced into a single pressure container. Liquid and gaseous propyne-containing recycle streams to be discussed hereinafter are also introduced into the pressure container. The reaction mixture passes the reactor at a LHSV of about 0.62 l/l/hr and is withdrawn in the form of a reactor effluent comprising 28.6% of the propyne unconverted.

The reactor effluent is flashed to 1.7 barabs and the resulting liquid stream is fed to a first distillation column to remove the remaining light ends from a bottom stream mainly comprising methyl methacrylate and the excess of methanol. The light ends over the top of the first distillation column are partially condensed at -18 °C in a first condenser. The first condenser liquid stream comprising 10.5 kg/hr propyne, 118.2 kg/hr methanol, 33.6 kg/hr methyl methacrylate and components as dimethylketone is bled. The first condenser vapour stream is compressed to 1.7 barabs in a first compressor and combined with the vapour stream from the reactor effluent flash. The combined vapour streams are compressed to 2.9 barabs in a second compressor, and partially condensed at -18 °C in a second condenser. The second condenser liquid stream comprising 534.6 kg/hr propyne, 162.8 kg/hr methanol, 39.2 kg/hr methyl methacrylate and 34.9 kg/hr carbon monoxide, is recycled to the reaction container. The second condenser vapour stream is partially bled, comprising 61.3 kg/hr propyne and 51.2 kg/hr carbon monoxide, besides inerts as nitrogen and hydrogen. The second condenser vapour stream comprising 165.6 kg/hr propyne and 138.4 kg/hr carbon monoxide, is multistage compressed with intercooling up to 12 barabs in a third compressor and recycled to the reaction container.

The bottom stream of the first distillation column is fed to a second distillation column, from which the vapour stream over the top is recycled to the first distillation column, a liquid azeotrope stream comprising methyl methacrylate and methanol, is recycled to the reaction container and a crude methyl methacrylate bottom stream is fed to a third distillation column.

A pure methyl methacrylate stream of 4728 kg/hr is produced over the top of the third distillation column. The heavy ends bottom stream is bled, comprising 17.5 kg/hr methyl methacrylate, 35.4 kg/hr methyl crotonate, catalyst residues and remaining heavy ends.

The overall conversion of propyne in the process is 96.4%, and 94.9% of the starting propyne is converted into methyl methacrylate product. Per 1000 kg of methyl methacrylate product, 15.1 kg propyne, 10.9 kg carbon monoxide and 25.0 kg methanol are lost.

It is seen that in the process of Example 3 outside the scope of the invention, a consumption of the precursors almost as efficient as in the Examples 1 and 2 according to the invention, can only be achieved when using a complicated recycle system comprising two condensers and three compressors. Apart from the investment costs for this equipment, it is will be appreciated that the large volumes of the recycle streams further adds utility costs.

## Claims

1. A continuous process for the carbonylation of an acetylenically unsaturated compound by reaction with carbon monoxide and a nucleophilic compound having a mobile hydrogen atom in the presence of a carbonylation catalyst, which process comprises the steps of:
a) charging said carbonylation catalyst to a reaction zone;
b) supplying a fresh feed containing said acetylenically unsaturated compound, said nucleophilic compound as well as carbon monoxide to said reaction zone;
c) passing the feed through the reaction zone under carbonylating conditions of pressure and temperature such that a degree of conversion, based on the acetylenically unsaturated compound, of at least 80% is effected;
d) separating a stream containing unreacted acetylenically unsaturated compound from the effluent of the reaction zone; and
e) recycling the unreacted acetylenically unsaturated compound content of said separated stream to the reaction zone.

2. A process according to claim 1, wherein the nucleophilic compound having a mobile hydrogen atom is a hydroxy compound.

3. A process according to claim 1 or 2, wherein an alkyl methacrylate is prepared by reaction of propyne with carbon monoxide and an alkanol.

4. A process according to any one or more of claims 1-3, comprising the further step of:
f) separating a stream containing unreacted nucleophilic compound having a mobile hydrogen atom from the effluent of the reaction zone for recycling to the reaction zone.

5. A process according to any one or more of claims 1-4, wherein a degree of conversion, based on the acetylenically unsaturated compound, of at least 90% is achieved in the reaction zone.

6. A process according to any one or more of claims 1-5, wherein the molar ratio of said acetylenically unsaturated compound to said nucleophilic compound is between 0.8 : 1 and 1 : 0.8.

7. A process according to claim 6, wherein said ratio is between 0.9 : 1 and 1 : 0.9.

8. A process according to any one or more of claims 1-7, wherein step d) is effected by stripping the effluent of the reaction zone with fresh carbon monoxide feed to the process.

9. A process according to claim 8, wherein said stripping is effected in a stripping zone operated at the pressure of the fresh carbon monoxide feed, preferably a pressure in the range of 5 - 20 barabs.

10. A process according to any one or more of claims 8-9, wherein said stripping is effected at a temperature in the range of 20 - 100 °C.

## Patentansprüche

1. Kontinuierliches Verfahren zur Carbonylierung einer acetylenisch ungesättigten Verbindung durch Umsetzung mit Kohlenmonoxid und einer nucleophilen Verbindung mit einem beweglichen Wasserstoffatom in Gegenwart eines Carbonylierungskatalysators, bei dem man:
a) den Carbonylierungskatalysator in eine Reaktionszone einbringt;
b) der Reaktionszone frischen, die acetylenisch ungesättigte Verbindung, die nucleophile Verbindung sowie Kohlenmonoxid enthaltenden Einsatzstoff zuführt;
c) den Einsatzstoff unter solchen Carbonylierungsbedingungen bezüglich Druck und Temperatur durch die Reaktionszone leitet, daß ein Umsatz von mindestens 80%, bezogen auf die acetylenisch ungesättigte Verbindung, erzielt wird;
d) aus dem Austragsstrom der Reaktionszone einen nicht umgesetzte acetylenisch ungesättigte Verbindung enthaltenden Strom abtrennt und
e) den Gehalt des abgetrennten Stroms an nicht umgesetzter acetylenisch ungesättigter Verbindung in die Reaktionszone zurückführt.

2. Verfahren nach Anspruch 1, bei dem man als nucleophile Verbindung mit einem beweglichen Wasserstoffatom eine Hydroxyverbindung einsetzt.

3. Verfahren nach Anspruch 1 oder 2, bei dem man durch Umsetzung von Propin mit Kohlenmonoxid und einem Alkanol ein Alkylmethacrylat herstellt.

4. Verfahren nach einem oder mehreren der Ansprüche 1-3, bei dem man weiter:
f) aus dem Austragsstrom der Reaktionszone einen nicht umgesetzte nucleophile Verbindung mit einem beweglichen Wasserstoffatom enthaltenden Strom zur Rückführung in die Reaktionszone abtrennt.

5. Verfahren nach einem oder mehreren der Ansprüche 1-4, bei dem man in der Reaktionszone einen Umsatz von mindestens 90%, bezogen auf die acetylenisch ungesättigte Verbindung, erzielt.

6. Verfahren nach einem oder mehreren der Ansprüche 1-5, bei dem man ein Molverhältnis der acetylenisch ungesättigten Verbindung zur nucleophilen Verbindung zwischen 0,8 : 1 und 1 : 0,8 wählt.

7. Verfahren nach Anspruch 6, bei dem man ein Verhältnis zwischen 0,9 : 1 und 1 : 0,9 wählt.

8. Verfahren nach einem der Ansprüche 1-7, bei dem man Schritt d) durch Strippen des Austragsstroms der Reaktionszone mit frischem Kohlenmonoxid-Einsatzstoff durchführt.

9. Verfahren nach Anspruch 8, bei dem man das Strippen in einer Strippzone durchführt, die beim Druck des frischen Kohlenmonoxid-Einsatzstoffs, bevorzugt bei einem Druck im Bereich von 5 - 20 bar abs., arbeitet.

10. Verfahren nach einem oder beiden der Ansprüche 8-9, bei dem man das Strippen bei einer Temperatur im Bereich von 20 - 100°C durchführt.

## Revendications

1. Procédé continu pour la carbonylation d'un composé acétyléniquement insaturé par réaction avec le monoxyde de carbone et un composé nucléophile ayant un atome d'hydrogène mobile en présence d'un catalyseur de carbonylation, lequel procédé comprend les étapes:
a) de chargement dudit catalyseur de carbonylation dans la zone de réaction;
b) de fourniture d'une alimentation fraîche contenant ledit composé acétyléniquement insaturé, ledit composé nucléophile ainsi que le monoxyde de carbone à ladite zone de réaction;
c) de passage de l'alimentation à travers la zone de réaction dans des conditions de carbonylation en pression et en température telles qu'un degré de conversion, basé sur le composé acétyléniquement insaturé, d'au moins 80 % est réalisé;
d) de séparation d'un courant, contenant du composé acétyléniquement insaturé, n'ayant pas réagi, de l'effluent de la zone de réaction; et
e) de recyclage de la teneur du composé, acétyléniquement insaturé, n'ayant pas réagi, dudit courant séparé dans la zone de réaction.

2. Procédé selon la revendication 1, caractérisé en ce que le composé nucléophile ayant un atome d'hydrogène mobile est un composé hydroxyle.

3. Procédé selon la revendication 1 ou 2, caractérisé en ce que l'on prépare un méthacrylate d'alkyle par réaction du propyne avec le monoxyde de carbone et un alcanol.

4. Procédé selon l'une quelconque des revendications 1 - 3, comprenant l'étape supplémentaire:
f) de séparation d'un courant contenant du composé nucléophile, n'ayant pas réagi, ayant un atome d'hydrogène mobile, de l'effluent de la zone de réaction pour le recyclage dans la zone de réaction.

5. Procédé selon l'une quelconque des revendications 1-4, caractérisé en ce que l'on obtient dans la zone de réaction un degré de conversion, basé sur le composé acétyléniquement insaturé, d'au moins 90 %.

6. Procédé selon l'une quelconque des revendications 1-5, caractérisé en ce que le rapport molaire dudit composé acétyléniquement insaturé audit composé nucléophile est compris entre 0,8 : 1 et 1 : 0,8.

7. Procédé selon la revendication 6, caractérisé en ce que ledit rapport est compris entre 0,9 : 1 et 1 : 0,9.

8. Procédé selon l'une quelconque des revendications 1-7, caractérisé en ce que l'étape d) est effectuée par strippage de l'effluent de la zone de réaction à l'aide d'une alimentation en monoxyde de carbone frais au procédé.

9. Procédé selon la revendication 8, caractérisé en ce que ledit strippage est effectué dans une zone de strippage en opération à la pression de l'alimentation en monoxyde de carbone frais, de préférence à une pression dans le domaine de 5 - 20 bar en pression absolue.

10. Procédé selon l'une quelconque des revendications 8-9, caractérisé en ce que ledit strippage est effectué à une température dans le domaine de 20 - 100 C.
